# EUROPEAN PATENT SPECIFICATION

(11) **EP 2 421 380 B1**
(45) Date of publication and mention of the grant of the patent: **23.08.2017**
(21) Application number: 10721365.4
(22) Date of filing: 23.04.2010
(51) Int. Cl.: A61P 1/16, A23C 7/04, A23C 15/14, A23L 33/12, A23C 13/12, A23C 9/20

(54) **A REDUCED-CHOLESTEROL DAIRY PRODUCT FOR USE IN THE TREATMENT AND/OR PREVENTION OF A LIVER DISEASE**
MILCHPRODUKT MIT REDUZIERTEM CHOLESTERIN ZUR VERWENDUNG ZUR BEHANDLUNG UND PREVENTION EINER ERKRANKUNG DER LEBER
Produit laitier à teneur réduite en cholestérol destiné à etre utilisé dans le traitement et/ou la prévention de la maladie du foie.

(30) Priority: 23.04.2009 EP 09158666
(43) Date of publication of application: 29.02.2012
(73) Proprietor: S. A. Corman, 4834 Goe (BE)
(72) Inventor: DALEMANS, Daniel, B-4040 Herstal (BE)
(74) Representative: Pronovem
(86) International application number: PCT/EP2010/055424
(87) International publication number: WO 2010/122138

(56) References cited:
- EP-A- 0 387 708
- EP-A- 0 615 690
- WO-A-2004/052122
- WO-A-2009/056493
- WO-A1-2007/090289
- BE-A4- 862 264
- US-A- 4 996 072
- US-A- 5 092 964
- US-A- 5 340 602
- US-A1- 2003 198 728
- US-A1- 2004 191 294
- US-B1- 6 303 803
- J.J. KIM, T.H. JUNG, J. AHN, AND H.S. KWAK: "Properties of cholesterol-reduced butter made with beta-cyclodextrin and added evening primrose oil and phytosterols" JOURNAL OF DAIRY SCIENCE, vol. 89, 2006, pages 4503-4510, XP002530837
- GURUPREET ANAND: "New Low cholesterol Herbal Ghee (Clarified butter). Alert today. Alive tomorrow!!!" PRLOG.ORG - GLOBAL PRESS RELEASE DISTRIBUTION, [Online] 11 April 2007 (2007-04-11), pages 1-1, XP002530838 Retrieved from the Internet: URL:http://www.prlog.org/10013053-new-low- cholesterol-herbal-ghee-clarified-butter-a lert-today-alive-tomorrow.pdf> [retrieved on 2009-06-04]
- BENITO ET AL: "Effects of milk enriched with omega-3 fatty acid, oleic acid and folic acid in patients with metabolic syndrome" 1 August 2006 (2006-08-01), CLINICAL NUTRITION, CHURCHILL LIVINGSTONE, LONDON, GB, PAGE(S) 581 - 587 , XP005560059 ISSN: 0261-5614 page 582, column 1, paragraph 1 - column 2, paragraph 1; table 1
- ANONYMOUS: "Oil, vegetable, corn, industrial and retail, all purpose salad or cooking" USDA NATIONAL NUTRIENT DATABASE FOR STANDARD REFERENCE, RELEASE 21, [Online] 2008, pages 1-3, XP002530839 Retrieved from the Internet: URL:http://www.nal.usda.gov/fnic/foodcomp/ cgi-bin/list_nut_edit.pl> [retrieved on 2009-06-05]
- ANONYMOUS: "Oil, soybean, salad or cooking (1)" USDA NATIONAL NUTRIENT DATABASE FOR STANDARD REFERENCE, RELEASE 21, [Online] 2008, XP002530840 ONLINE Retrieved from the Internet: URL:http://www.nal.usda.gov/fnic/foodcomp/ cgi-bin/list_nut_edit.pl> [retrieved on 2009-06-05]
- ANONYMOUS: "Milk, whole, 3.25% milkfat" USDA NATIONAL NUTRIENT DATABASE FOR STANDARD REFERENCE, RELEASE 21, [Online] 2008, pages 1-3, XP002530848 Retrieved from the Internet: URL:http://www.nal.usda.gov/fnic/foodcomp/ cgi-bin/list_nut_edit.pl> [retrieved on 2009-06-04]
- MATTHEW SHULMAN: "Linking Alzheimer's and Cholesterol" US NEWS AND WORLD REPORT, [Online] 18 April 2008 (2008-04-18), pages 1-1, XP002530841 ONLINE Retrieved from the Internet: URL:http://health.usnews.com/articles/heal th/2008/04/18/linking-alzheimers-and-chole sterol.html> [retrieved on 2009-06-04]
- Anonymous: "Ghee - Wikipedia, the free encyclopedia", Wikipedia, 8 January 2016 (2016-01-08), pages 1-6, XP055242321, Retrieved from the Internet: URL:https://en.wikipedia.org/wiki/Ghee [retrieved on 2016-01-18]
- Anonymous: "Omega 3 Butter - Record ID: 351747", GNPD, 1 April 2005 (2005-04-01), pages 1-2, XP055242382, Retrieved from the Internet: URL:http://www.gnpd.com/sinatra/recordpage /351747/from_search/0LqSqrmGat/?page=1 [retrieved on 2016-01-18]
- Anonymous: "Kevytvoi Lättsmör Low Fat Butter - Record ID: 287418", GNPD, 1 July 2004 (2004-07-01), page 12, XP055242385, Retrieved from the Internet: URL:http://www.gnpd.com/sinatra/recordpage /287418/from_search/H65bwee7wh/?page=1 [retrieved on 2016-01-18]
- Anonymous: "Butter, without salt - 01154", USDA National Nutrient Database for Standard Reference Release 28, 1 May 2016 (2016-05-01), pages 1-5, XP055287227, Retrieved from the Internet: URL:https://ndb.nal.usda.gov/ndb/foods/sho w/132?fgcd=&manu=&lfacet=&format=&count=&m ax=50&offset=&sort=default&order=asc&qlook up=01145&ds=&qt=&qp=&qa=&qn=&q=&ing= [retrieved on 2016-07-11]

## Description

### Field of the invention

The present invention is in the field of therapy and/or prevention of mammal (especially human) liver disease and is related to a dairy product (milk or milk product) including food or feed compositions comprising this dairy product, this dairy product being obtained from a ruminant, preferably a cattle dairy product (milk or milk product), more preferably a cow dairy product (milk or milk product) with a reduced cholesterol content.

### Background of the invention.

There is a tendency in developed countries to consume food rich in triglycerides or fat (i.e. about 22 % of total intake; measured on dry weight) and in cholesterol.

Lipid disorder is a medical term to define high blood cholesterol and triglycerides mammal blood ratios, which is linked to the risk increase of cardiovascular (such as atherosclerosis) and other heart diseases in mammals, especially in humans.

Milk contains about 3 to about 8 % of fat (w:w), that is mainly a saturated fat.

Fat (triglycerides) is essential in milk for its flavor and for some of its 'technical' properties, especially in milk-derived products (dairy products), such as spreads, cheeses, creams, and so on.

Fat content in milk-derived product may be of about 3% to about 100% of dry weight (between about 10% and 80 % on total weight). For instance cheese and butter contains about 45% (expressed in dry weight) and about 80% of fat (expressed in total weight or up to 98% in dry weight), respectively.

Unsaturated fatty acids, especially ω3 poly-unsaturated fatty acids, such as α-linolenic acid (ALA), Eicosapentaenoic acid (EPA) and/or Docosahexaenoic acid (DHA) appear to exert positive health effects and their properties are presented in functional food compositions, food complements or nutraceuticals enriched in these poly-unsaturated fats.

These specific fatty acids may be present in the form of fat (triglycerides), phospholipids, or as fatty acids conjugated to carriers, such as lipoproteins.

However, (ruminant) milk is poor on (poly-) unsaturated fatty acids. For regulatory reasons, a dairy product (milk product) enriched with exogenous fatty acids (for instance by adding vegetal fat) may not be labeled as a dairy product (milk-product).

Milk fat further contains cholesterol (about 300 mg for 100 g of fat). However, cholesterol food intake should be limited to a maximum of 300 mg per day. Consequently, improving the nutritional image of milk fat involves the reduction of its cholesterol content.

Both cholesterol and saturated fat are known to adversely affect (human) health, especially with regard to cardiovascular diseases and to some cancers.

Therefore, whole milk, milk fat and dairy products, which are rich in this milk fat (such as spreads, creams, cheeses...) and any food or feed compositions comprising these dairy products suffer from a bad nutritional image, because of both their high saturated fatty acids and their high cholesterol contents linked to these health issues.

### State of the art.

Presently, extraction of cholesterol from milk fat is achieved industrially by physical methods, such as molecular encapsulation by contact with a cyclodextrin (EP-387708 B1) or by steam stripping. With these methods, a minimum of 75% of the initially present cholesterol may be extracted.

Alternatively, several methods were developed to reduce cholesterol content in milk, or its bio-availability.

For instance US 6 485 931 B2 describes milk-cholesterol conversion into coprostanol, with a lower availability.

The international patent application WO 2004 052 122 describes methods to reduce the cholesterol content of animal-derived fat by changing their regimen.

The European patent EP 1585508 B1 is related to the use of sphingolipids for the manufacture of a food or pharmaceutical product for lowering cholesterol and triglyceride in plasma and/or serum and to the use of sphingolipids for the treatment or prevention of cardiovascular diseases in humans through lowering cholesterol and triglyceride in plasma and/or serum.

EP 0615 690 describes the extraction of milk-cholesterol using oils and US 5 175 015 describes a skimmed milk mixed with vegetable fat.

WO2007/090289 shows that berberine chloride and a plant stanol either alone or in combination, dramatically reduced cholesterol concentration in the liver of hamsters and could be used to treat fatty liver.

### Aims of the invention

The present invention aims to provide a food or feed element or a food or feed composition that does not present the drawbacks of the state of the art, especially an element or composition that may prevent and/or treat a liver disease.

The present invention aims to provide such element or composition that can be used as a functional food or feed (composition) or as a nutraceutical and that can be presented in usual dairy products or in feed or food compositions comprising these dairy products (milk and milk beverages, spreads, especially butter, creams, cheese, etc) or obtained from these dairy products (ice creams, bakery and confectionery products, etc)

### Summary of the invention

The Present invention is related to a cholesterol-reduced ruminant dairy product for use in the treatment and/or the prevention of a liver disease, preferably steatosis, the said dairy product (milk or milk product), containing from 60% to 70% of saturated fat (w _{saturated fat}: w_{total fat}) wherein the cholesterol content is comprised between 30 mg/100 g fat and 150 mg/100 g fat, preferably between 30mg/100g fat and 90mg/100gfat.

Preferably the cholesterol-reduced ruminant dairy product (milk or milk product), of the invention is obtained from a cow.

The present invention also relates to a cholesterol-reduced ruminant dairy product, for use in the treatment and/or prevention of a liver disease, wherein the said dairy product is supplemented with an ω3 fatty acid source selected from the group consisting of α-linolenic acid (ALA), Eicosapentaenoic acid (EPA) and Docosahexaenoic acid (DHA) (wherein ω3 fatty acids are added at 2 % (w_{ω3}:w_{fat}) to 10 % (w_{ω3}:w_{fat}), preferably at 2 % (w_{ω3}:w_{fat}) to 8 % (w_{ω3}:w_{fat}), preferably at 6% (w_{ω3}:w_{fat}) (i.e: compared to the total weight of the dairy product fat being 100%).

Advantageously, the dairy product of the invention is a milk that comprises between 3% and 4 % (w:w) of protein (of which about 80% of casein), between 3% and 6 % (w:w) of fat, between 4% and 5% (w:w) of carbohydrates and between 0.6 and 1% of minerals, being mainly calcium, potassium, chloride and phosphorus (i.e. compared to the total weight % of the dairy product being 100%).

Preferably, the cholesterol-reduced ruminant dairy product of the invention is selected from the group consisting of milk, anhydrous milk fat, spread, preferably a butter, ice cream, milk cream, cheese, fermented milk, flavoured milk and cream. The present invention is also related to a (functional) food or feed composition comprising the dairy product or the dairy fat of the invention or obtained from the dairy product or dairy fat of the invention, such as ice cream, bakery or confectionary products.

By 'about', it is preferably meant every real number plus or minus 10%. For example by 'about 4%', it is meant every real numbers between 3.6% and 4.4%.

The ruminant dairy product (milk, milk product or fat) of the present invention and obtained from the ruminant milk, may be used alone (as milk, a milk cream or anhydrous milk fat), as a derived product (as a spread like butter, a milk beverage or cheese), or as a supplement or active ingredient in a (functional) feed composition or in a (functional) food composition with the other usual ingredients of this composition, as or in a nutraceutical composition, and/or as or in a pharmaceutical composition.

Advantageously, this medicament (pharmaceutical composition) and/or nutraceutical comprising the dairy product, especially the dairy fat, of the invention is provided (present) in a food composition, preferably a spread, more preferably a butter having between 10% and 50% (preferably of about 40%) (w:w) total fat (lipid) and a cholesterol content comprised between 30 mg/100 g fat and 150 mg/100 g fat, more preferably between 30 mg/100 fat and 90 mg/100 g fat.

The consumption of the dairy product, especially the dairy fat, of the present invention advantageously increases (long chain) poly-unsaturated fatty acids content (such as Arachidonic acid, Eicosapentaenoic acid and Docosahexaenoic acid) in a mammal (including the human) blood.

More precisely, the consumption of dairy product, especially the dairy fat, of the present invention further increases long chain poly-unsaturated ω3 fatty acids content in a mammal (including the human) blood.

By long chain ω3 fatty acids, it is meant Eicosapentaenoic acid (EPA) and/or Docosahexaenoic acid (DHA).

The consumption of the dairy product, especially the dairy fat, of the invention further decreases the triglyceride content in a mammal (including the human) blood (compared to a ruminant milk fat that does not have a reduced cholesterol content (having a cholesterol content of about 280 mg/100 g fat)).

The consumption of the dairy product, especially the dairy fat, of the invention increases by at least 15% (preferably by at least 30%, more preferably by at least 50%) the HDL-cholesterol content in a mammal (including the human) blood and preferably increases non-HDL blood cholesterol content by less than 10%, more preferably by less than 20%.

Therefore, the consumption of the dairy product, especially the dairy fat, of the present invention also decreases the atherogenic ratio of mammal (including a human) blood by at least 10%, preferably by at least 20%, more preferably by at least 30%.

### Detailed description of the invention

As described in the method of the European patent EP-387708 B1, the inventors reduced the cholesterol content of cow milk by treating it with β-cyclodextrin.

More precisely, 5 kg cow milk fat containing 278 mg cholesterol/100 g fat was mixed during 30 minutes at 55°C with 5 kg of a 6% beta-cyclodextrin solution to form a "oil in water" emulsion. Milk fat with a lower content of cholesterol (25 mg/100 g) was recovered by continuous centrifugation, washing with water, second continuous centrifugation and drying under vacuum.

The inventors treated 56 hamster animals, being a cardiovascular model adapted to the human population with several regimens that mimics human diets (regarding fat content of 12.5% and 22% (% on dry weight)) and containing milk fat treated or not and with a classical (milk fat free) regimen.

By comparing animals fed with regimen (heavily) enriched with milk fat and classical regimen, the inventors observed a consistent increase in blood cholesterol and a tendency of increase in blood triglycerides. However, atherogenic ratio, being the HDL/non-HDL cholesterol ratio was only marginally impaired.

Therefore, the inventors concluded that feeding an animal (including a human) with a cholesterol-rich milk fat (or untreated, i.e. containing about 280 mg cholesterol per 100 g fat) results in the worsening of most of blood parameters with regard to a cardiovascular risk and associated diseases, consistent with the bad nutricional image of dairy products.

Furthermore, the inventors observed that the liver of hamsters fed with the cholesterol-rich milk fat is bigger, as well as their cholesterol and triglyceride contents, which are increased by about 6-fold and by about 2-fold.

The inventors measured in parallel the same parameters for hamsters fed with the reduced cholesterol milk fat (25 mg/100 g) of the invention.

By comparison with hamsters fed with cholesterol-rich milk fat, the inventors measured a consistent reduction in blood cholesterol, in triglycerides and in atherogenic ratio in animals fed with the reduced cholesterol fat of the invention.

The inventors conclude therefore that (all) the major blood parameters relevant to a measure of a cardiovascular risk and associated diseases are improved by the dairy product of the invention (reduced-cholesterol milk fat) by comparison to animals fed with normal milk fat (cholesterol rich).

By comparison with hamsters fed with the classical regimen, blood triglyceride content is not consistently improved in animals fed with the reduced-cholesterol milk fat of the invention.

Interestingly, by feeding animals with reduced-cholesterol milk fat (instead of the classical regimen), the 'bad' (non-HDL) cholesterol did not increase, while the good (HDL) cholesterol consistently increased, especially when giving regimens (comprising the dairy fat of the invention) at 22% of lipids, that mimic current Human diets, especially in Europe, USA and Japan.

The liver parameters such as total weight and cholesterol content (of animals fed with the dairy fat of the invention) are similar to those of hamsters fed with classical regimen (not comprising milk fat), with the exception of triglyceride and phospholipid contents that were higher.

In all cases, the atherogenic ratio is consistently and significantly decreased in animals fed with the reduced-cholesterol milk fat of the invention.

The inventors therefore concluded that, although they fed animals with a product that is rich in saturated fat, their blood and liver parameters were not degraded and, in some aspects, they were improved.

Further refining their observations, the inventors measured a consistent and significant increase of EPA and DHA plasma contents in animals fed with the reduced-cholesterol milk of the invention.

Since there is a need to increase the blood content of long chain ω3 fatty acids in animals (including humans), the inventors deduce that the low-cholesterol milk fat they tested is a very suitable nutraceutical, functional feed (or food) composition or an adequate and efficient pharmaceutical ingredient or composition.

The inventors conclude that, in long terms, this reduced-cholesterol milk fat is safe and even improves several blood parameters of an mammal (including a human).

The inventors further fed other group of hamsters with milk fat enriched with ω3 essential fatty acid α-linolenic acid (ALA).

With the exception of a strong increase in plasma content of the α-linolenic acid, the inventors noticed only a marginal reduction of triglycerides and cholesterol contents in their blood by comparison to control groups.

The inventors further noticed that both the addition to the milk fat of the ω3 fatty acid α-linolenic acid and the reduction of the cholesterol content of this milk fat (enriched in ALA) synergize and provoke a strong reduction of the blood cholesterol and triglyceride contents, coupled with a consistent increase of the plasma content of the three ω3 fatty acids measured (ALA, EPA and DHA).

Therefore, although one may fear that an animal (including a human) fed with low-cholesterol milk (fat) will have increased blood and liver triglyceride values (and will be at higher cardiovascular risk), these comparative data clearly shows that this feared increase is not necessary spectacular or consistent and does not translate into an increase of the liver size. More importantly, the reduced-cholesterol milk fat of the invention results in an increase of long chain ω3 fatty acids, such as EPA and DHA measured in the mammal plasma, these long chain ω3 fatty acids being known to improve several health related aspects.
The present invention will be described in more details in the following non limiting examples.

The inventors developed a regimen whereby milk fat is replaced by low-cholesterol milk fat and/or regimen enriched in low-cholesterol milk-fat.

A typical regimen contains from about 10 to about 22% of total fat (w:w; dry weight) and a reduced cholesterol content (comprised between about 10 mg/100 g (total) fat and about 150 mg/100 g (total) fat, preferably comprised between about 30 mg/100 g (total) fat and about 150 mg/100 g (total) fat, more preferably comprised between about 30 mg/100 (total) fat and about 90 mg/100 g (total) fat).

Alternatively, the skilled person and/or the consumer may replicate such a regimen by replacing dairy products usually taken (and possibly by reducing its consumption of other cholesterol-rich products such as meat) by the dairy products of the invention (that contain the reduced-cholesterol milk fat of the invention).

### Examples

### Example 1

The inventors divided 56 hamsters in 8 groups.
- Group G1: hamsters fed with fat from winter milk.
- Group G2: hamsters fed with fat from winter milk further depleted in cholesterol (i.e. low-cholesterol milk-fat).
- Group G3: hamsters fed with fat from spring milk.
- Group G4: hamsters fed with fat from spring milk further enriched in ALA.
- Group G5: hamsters fed with fat from spring milk further strongly enriched in ALA.
- Group G6: hamsters fed with fat from spring milk further depleted in cholesterol and enriched in ALA.
- Group G7: hamsters fed with fat from spring milk further depleted in cholesterol and strongly enriched in ALA.
- Group G8: control hamsters having received classical feed.

In order to deplete cholesterol, the inventors mixed milk fat with β-cyclodextrin. The cholesterol content was reduced by about 90%.

The hamsters were given for 5 weeks a first regimen having 12.5% of lipids (further characterized on Table 1 below).

**Table 1: 12.5% lipid diet**

| | **G1** | **G2** | **G3** | **G4** | **G5** | **G6** | **G7** |
|---|---|---|---|---|---|---|---|
| **Dry food (100g)** | | | | | | | |
| starch (g) | 37,50 | 37,50 | 37,50 | 37,50 | 37,50 | 37,50 | 37,50 |
| sucrose (g) | 17,50 | 17,50 | 17,50 | 17,50 | 17,50 | 17,50 | 17,50 |
| casein (g) | 20,00 | 20,00 | 20,00 | 20,00 | 20,00 | 20,00 | 20,00 |
| total lipid(g) | 12,50 | 12,50 | 12,50 | 12,50 | 12,50 | 12,50 | 12,50 |
| saturated (g) | 8,59 | 8,55 | 7,80 | 7,60 | 6,94 | 7,55 | 6,92 |
| LA (g) | 0,20 | 0,20 | 0,32 | 0,39 | 0,58 | 0,40 | 0,58 |
| ALA (g) | 0,08 | 0,08 | 0,10 | 0,37 | 0,95 | 0,36 | 0,96 |
| LA/ALA | 2,42 | 2,52 | 3,09 | 1,07 | 0,61 | 1,11 | 0,60 |
| cholesterol (mg) | 34,8 | 3,1 | 32,0 | 31,9 | 27,8 | 3,8 | 3,3 |
| cellulose (g) | 5,00 | 5,00 | 5,00 | 5,00 | 5,00 | 5,00 | 5,00 |
| minerals (g) | 5,00 | 5,00 | 5,00 | 5,00 | 5,00 | 5,00 | 5,00 |
| vitamins (g) | 2,50 | 2,50 | 2,50 | 2,50 | 2,50 | 2,50 | 2,50 |
| | | | | | | | |
| **Total energy intake (%)** | | | | | | | |
| sugar | 53,30 | 53,30 | 53,30 | 53,30 | 53,30 | 53,30 | 53,30 |
| protein | 19,40 | 19,40 | 19,40 | 19,40 | 19,40 | 19,40 | 19,40 |
| total lipid(g) | 27,30 | 27,30 | 27,30 | 27,30 | 27,30 | 27,30 | 27,30 |
| saturated | 18,75 | 18,66 | 17,03 | 16,57 | 15,15 | 16,47 | 15,09 |
| LA | 0,43 | 0,43 | 0,69 | 0,85 | 1,25 | 0,87 | 1,26 |
| ALA | 0,18 | 0,17 | 0,22 | 0,80 | 2,07 | 0,78 | 2,08 |

After 5 weeks, blood samples were collected by cardiac puncture on starved animals.

Thereafter, the hamsters were given for 12 weeks a regimen having 22% of lipids (further characterized on Table 2 below).

**Table 2: 22% lipid diet**

| | G1 | G2 | G3 | G4 | G5 | G6 | G7 |
|---|---|---|---|---|---|---|---|
| **Dry food (100g)** | | | | | | | |
| starch (g) | 37,50 | 37,50 | 37,50 | 37,50 | 37,50 | 37,50 | 37,50 |
| sucrose (g) | 8,00 | 8,00 | 8,00 | 8,00 | 8,00 | 8,00 | 8,00 |
| casein (g) | 20,00 | 20,00 | 20,00 | 20,00 | 20,00 | 20,00 | 20,00 |
| total lipid(g) | 22,00 | 22,00 | 22,00 | 22,00 | 22,00 | 22,00 | 22,00 |
| saturated (g) | 15,12 | 15,05 | 13,73 | 13,37 | 12,22 | 13,29 | 12,17 |
| LA (g) | 0,35 | 0,35 | 0,56 | 0,69 | 1,01 | 0,70 | 1,02 |
| ALA (g) | 0,14 | 0,14 | 0,18 | 0,64 | 1,67 | 0,63 | 1,68 |
| LA/ALA | 2,42 | 2,52 | 3,09 | 1,07 | 0,61 | 1,11 | 0,60 |
| cholesterol (mg) | 61,2 | 5,5 | 56,3 | 56,1 | 48,8 | 6,6 | 5,7 |
| cellulose (g) | 5,00 | 5,00 | 5,00 | 5,00 | 5,00 | 5,00 | 5,00 |
| minerals (g) | 5,00 | 5,00 | 5,00 | 5,00 | 5,00 | 5,00 | 5,00 |
| vitamins (g) | 2,50 | 2,50 | 2,50 | 2,50 | 2,50 | 2,50 | 2,50 |
| | | | | | | | |

| **Total energy intake (%)** | | | | | | | |
|---|---|---|---|---|---|---|---|
| sugar | 39,60 | 39,60 | 39,60 | 39,60 | 39,60 | 39,60 | 39,60 |
| protein | 17,40 | 17,40 | 17,40 | 17,40 | 17,40 | 17,40 | 17,40 |
| total lipid(g) | 43,00 | 43,00 | 43,00 | 43,00 | 43,00 | 43,00 | 43,00 |
| saturated | 29,59 | 29,45 | 26,87 | 26,15 | 23,91 | 25,99 | 23,82 |
| LA | 0,68 | 0,68 | 1,09 | 1,35 | 1,98 | 1,37 | 1,99 |
| ALA | 0,28 | 0,27 | 0,35 | 1,26 | 3,26 | 1,24 | 3,29 |

Note: in Tables 1 and 2, minerals and vitamins are provided in a mix. Standard mixes provide the recommended daily allowance of minerals and vitamins. LA stands for linoleic acid (ω6 essential fatty acid).

Fifteen weeks after the beginning of the experiment (10 weeks after the 22% lipid regimen was given), a second blood sample was collected by cardiac puncture on starved animals.

Seventeen weeks after the beginning of the experiment (12 weeks after the 22% lipid regimen was given), a third blood sample was collected by cardiac puncture on animals having retained free access to their feed.

The animals were then sacrified in order to analyze their organs.

The plasma parameters are further depicted in Table 3.

**Table 3: Blood parameters at three times points.**

| Blood samples at week 5 | | | | | | | | |
|---|---|---|---|---|---|---|---|---|
| | **G1** | **G2** | **G3** | **G4** | **G5** | **G6** | **G7** | **G8** |
| Cholesterol (mg/dl) | 290 | 274 | 283 | 286 | 265 | 255 | 241 | 149 |
| Triglycerides (mg/dl) | 255 | 203 | 255 | 238 | 276 | 282 | 233 | 132 |
| Phospholipids (mg/dl) | 420 | 419 | 438 | 437 | 421 | 410 | 394 | 256 |
| HDL Cholesterol (mg/dl) | 174 | 186 | 180 | 189 | 148 | 157 | 151 | 78 |
| Non-HDL Cholesterol (mg/dl) | 116 | 90 | 103 | 97 | 117 | 99 | 90 | 69 |
| Non-HDL/HDL ratio | 0,80 | 0,49 | 0,58 | 0,54 | 0,83 | 0,66 | 0,60 | 0,89 |
| | | | | | | | | |

| Blood sample at week 15 | | | | | | | | |
|---|---|---|---|---|---|---|---|---|
| | **G1** | **G2** | **G3** | **G4** | **G5** | **G6** | **G7** | **G8** |
| Cholesterol (mg/dl) | 285 | 224 | 283 | 254 | 225 | 212 | 162 | 133 |
| Triglycerides (mg/dl) | 272 | 121 | 260 | 188 | 216 | 174 | 164 | 105 |
| Phospholipids (mg/dl) | 436 | 345 | 435 | 393 | 337 | 329 | 281 | 227 |
| HDL Cholesterol (mg/dl) | 184 | 150 | 182 | 172 | 135 | 131 | 101 | 82 |
| Non-HDL Cholesterol (mg/dl) | 101 | 79 | 101 | 83 | 90 | 81 | 60 | 50 |
| Non-HDL/HDL ratio | 0,56 | 0,52 | 0,58 | 0,62 | 0,68 | 0,78 | 0,74 | 0,64 |
| | | | | | | | | |

| Blood sample at week 17 | | | | | | | | |
|---|---|---|---|---|---|---|---|---|
| | **G1** | **G2** | **G3** | **G4** | **G5** | **G6** | **G7** | **G8** |
| Cholesterol (mg/dl) | 257 | 214 | 256 | 242 | 226 | 208 | 159 | 226 |
| Triglycerides (mg/dl) | 275 | 242 | 252 | 256 | 310 | 219 | 214 | 310 |
| Phospholipids (mg/dl) | 424 | 396 | 419 | 399 | 363 | 376 | 325 | 363 |
| HDL Cholesterol (mg/dl) | 156 | 135 | 162 | 145 | 112 | 117 | 95 | 112 |
| Non-HDL Cholesterol (mg/dl) | 101 | 79 | 93 | 97 | 114 | 91 | 63 | 114 |
| Non-HDL/HDL ratio | 0,69 | 0,61 | 0,61 | 0,68 | 1,08 | 0,82 | 0,70 | 1,08 |

Non-HDL/HDL ratio stands for the atherogenic ratio measured as Non-HDL cholesterol divided by HDL cholesterol values. High atherogenic ratios represent an increased risk of cardiovascular attack.

Besides total cholesterol, cholesterol and saturated fatty acid intakes, the measured blood atherogenic ratio is an independent measure predictive of the risk of cardiovascular diseases.

**Table 4: animal weight and liver parameters**

| | **G1** | **G2** | **G3** | **G4** | **G5** | **G6** | **G7** | **G8** |
|---|---|---|---|---|---|---|---|---|
| Initial weight (g) | 56,8 | 57,8 | 56,1 | 56,5 | 56,8 | 56,4 | 55,3 | 58,2 |
| Weight at week 17 | 127,0 | 128,0 | 126,0 | 128,7 | 125,0 | 136,0 | 129,5 | 113,3 |
| Difference (g) | **70,3** | **70,3** | **69,9** | **72,0** | **68,3** | **78,7** | **74,3** | 55,2 |
| liver weight (g) | **6,45** | 5,76 | **6,45** | **6,64** | **6,19** | **6,23** | 5,72 | 4,66 |
| *SD* | *0,81* | *0,69* | *0,55* | *0,78* | *0,44* | *0,59* | *0,69* | *0,42* |
| liver/total %(w:w) | **5,07** | 4,5 | **5,11** | **5,15** | **4,97** | 4,58 | 4,42 | 4,11 |
| Chol (mg/g liver) | **19,39** | 3,98 | **25,96** | **20,13** | **12,65** | 4,47 | 3,03 | 3,55 |
| *SD* | *8,36* | *2,17* | *8,92* | *7,99* | *7,18* | *2,51* | *1,59* | *1,16* |

As depicted in Table 4, it is clear that the liver weight and its cholesterol content of groups receiving low-cholesterol milk fat regimens (G2 and G7) are close to the values of the control group (G8) or even improved over the control groups G1, G3 and for some aspects of G8.

In addition, the inventors measured plasma values for the fatty acids of all the hamsters in G1 to G7

**Table 5: Plasma values of fatty acids in groups 1 to 7**

| | **G1** | **G2** | **G3** | **G4** | **G5** | **G6** | **G7** |
|---|---|---|---|---|---|---|---|
| Triglycerides (% FA) | | | | | | | |
| Saturated | 60,58 | 58,58 | 48,68 | **46,54** | **45,71** | 50,64 | 47,02 |
| Mono unsaturated | **35,77** | 37,61 | 47,19 | 47,38 | 44,43 | 43,55 | 42,17 |
| poly unsaturated | 3,65 | **3,81** | 4,12 | 6,08 | **9,86** | **5,82** | **10,80** |
| EPA+DHA | 0,16 | **0,21** | 0,10 | 0,11 | **0,17** | **0,21** | 0,19 |
| | | | | | | | |

| Phospholipids (% FA) | | | | | | | |
|---|---|---|---|---|---|---|---|
| Saturated | 45,47 | 45,31 | 45,79 | **48,61** | **44,78** | 45,41 | 45,28 |
| Mono unsaturated | **36,04** | 32,51 | 33,15 | 30,66 | 27,22 | 27,58 | 22,95 |
| poly unsaturated | 18,49 | **22,18** | 21,05 | 20,73 | **28,00** | **27,01** | **31,77** |
| EPA+DHA | 2,92 | **4,36** | 2,33 | 1,58 | 2,83 | **3,65** | 3,96 |

As demonstrated in Table 5, the reduction of cholesterol in the fat given to the animals results into an increased poly-unsaturated fatty acids content in the plasma, at the expense of the mono-unsaturated fatty acid content for fatty acids in the form of phospholipids, and at the expense of both saturated and mono-unsaturated fatty acids for fatty acids in the form of triglycerides.

The addition of ALA in the regimen results into an increase of plasma poly-unsaturated fatty acids, but EPA and DHA values are not comparatively increased, and even failed to achieve bigger values as obtained in G2.

## Claims

1. A cholesterol-reduced ruminant dairy product for use in the treatment and/or prevention of a liver disease, the said product containing from 60% to 70% of saturated fat (w saturated fat : w total fat), wherein the cholesterol content is comprised between 30 mg/100g fat and 150 mg/100 g fat,.

2. The cholesterol-reduced ruminant dairy product for use in the treatment and/or the prevention of a liver disease according to claim 1, wherein the liver disease is steatosis.

3. The cholesterol-reduced ruminant dairy product for use in the treatment and /or the prevention of a liver disease according to claim 1, wherein the cholesterol content is comprised between 30 mg/100g fat and 90 mg/100 g fat.

4. The cholesterol-reduced ruminant dairy product according to claim 1 or 2, wherein the cholesterol-reduced ruminant dairy product is supplemented with an omega-3 fatty acid source selected from the group consisting of α-linoleic acid (ALA), Eicosapentaenoic acid (EPA) and Docosahexaenoic acid (DHA) and wherein the omega-3 fatty acid is added at 2 % (w_{ω3}:w_{fat}) to 10 % (w_{ω3}:w_{fat}).

5. The cholesterol-reduced ruminant dairy product for use in the treatment and/or the prevention of a liver disease according to any one of the preceding claims, wherein the cholesterol-reduced ruminant dairy product is selected from the group consisting of milk, anhydrous milk fat, spread, ice cream, milk cream, cheese, fermented milk, flavored milk, and cream.

6. The cholesterol-reduced ruminant dairy product for use in the treatment and/or the prevention of a liver disease according to claim 5 wherein the spread is butter or a spreadable butter.

## Patentansprüche

1. Cholesterinreduziertes Milchprodukt von Wiederkäuern zur Verwendung bei der Behandlung und/oder Verhütung einer Lebererkrankung, wobei das Produkt 60 % bis 70 % gesättigtes Fett (w gesättigtes Fett:w Gesamtfett) enthält, wobei der Cholesteringehalt zwischen 30 mg/100 g Fett und 150 mg/100 g Fett liegt.

2. Cholesterinreduziertes Milchprodukt von Wiederkäuern zur Verwendung bei der Behandlung und/oder Verhütung einer Lebererkrankung nach Anspruch 1, wobei es sich bei der Lebererkrankung um Steatose handelt.

3. Cholesterinreduziertes Milchprodukt von Wiederkäuern zur Verwendung bei der Behandlung und/oder Verhütung einer Lebererkrankung nach Anspruch 1, wobei der Cholesteringehalt zwischen 30 mg/100 g Fett und 90 mg/100 g Fett liegt.

4. Cholesterinreduziertes Milchprodukt von Wiederkäuern nach Anspruch 1 oder 2, wobei das cholesterinreduzierte Milchprodukt von Wiederkäuern mit einer Omega-3-Fettsäurequelle ergänzt ist, die aus der Gruppe bestehend aus α-Linolsäure (ALA), Eicosapentaensäure (EPA) und Docosahexaensäure (DHA) ausgewählt ist, und wobei die Omega-3-Fettsäure bei 2 % (w_{ω3}:w_{Fett}) bis 10 % (w_{ω3}:w_{Fett}) zugegeben wird.

5. Cholesterinreduziertes Milchprodukt von Wiederkäuern zur Verwendung bei der Behandlung und/oder Verhütung einer Lebererkrankung nach einem der vorhergehenden Ansprüche, wobei das cholesterinreduzierte Milchprodukt von Wiederkäuern aus der Gruppe bestehend aus Milch, wasserfreiem Milchfett, Aufstrich, Eiscreme, Milchrahm, Käse, Sauermilch, Milchmischgetränk und Sahne ausgewählt ist.

6. Cholesterinreduziertes Milchprodukt von Wiederkäuern zur Verwendung bei der Behandlung und/oder Verhütung einer Lebererkrankung nach Anspruch 5, wobei es sich bei dem Aufstrich um Butter oder eine streichfähige Butter handelt.

## Revendications

1. Produit laitier de ruminant à teneur réduite en cholestérol pour une utilisation dans le traitement et/ou la prévention d'une maladie hépatique, ledit produit contenant de 60 % à 70 % de graisses saturées (poids de graisses saturées : poids de graisses totales), dans lequel la teneur en cholestérol est comprise entre 30 mg/100 g de graisses et 150 mg/100 g de graisses.

2. Produit laitier de ruminant à teneur réduite en cholestérol pour une utilisation dans le traitement et/ou la prévention d'une maladie hépatique selon la revendication 1, dans lequel la maladie hépatique est la stéatose.

3. Produit laitier de ruminant à teneur réduite en cholestérol pour une utilisation dans le traitement et/ou la prévention d'une maladie hépatique selon la revendication 1, dans lequel la teneur en cholestérol est comprise entre 30 mg/100 g de graisses et 90 mg/100 g de graisses.

4. Produit laitier de ruminant à teneur réduite en cholestérol selon la revendication 1 ou 2, dans lequel le produit laitier de ruminant à teneur réduite en cholestérol est enrichi avec une source d'acide gras oméga-3 choisie dans le groupe constitué par l'acide α-linoléique (ALA), l'acide éicosapentaénoïque (EPA) et l'acide docosahexaénoïque (DHA) et dans lequel l'acide gras oméga-3 est ajouté à hauteur de 2 % (poids_{ω3}:poids_{graisses}) à 10 % (poids_{ω3}:poids_{graisses}).

5. Produit laitier de ruminant à teneur réduite en cholestérol pour une utilisation dans le traitement et/ou la prévention d'une maladie hépatique selon l'une quelconque des revendications précédentes, dans lequel le produit laitier de ruminant à teneur réduite en cholestérol est choisi dans le groupe constitué par le lait, les matières grasses de lait anhydres, une pâte à tartiner, une crème glacée, une crème de lait, un fromage, un lait fermenté, un lait aromatisé et une crème.

6. Produit laitier de ruminant à teneur réduite en cholestérol pour une utilisation dans le traitement et/ou la prévention d'une maladie hépatique selon la revendication 5, dans lequel la pâte à tartiner est du beurre ou un beurre à tartiner.
